# EUROPEAN PATENT SPECIFICATION

(11) **EP 0 697 887 B1**
(45) Date of publication and mention of the grant of the patent: **25.11.1998**
(21) Application number: 94916004.8
(22) Date of filing: 04.05.1994
(51) Int. Cl.: A61K 38/21, A61J 1/00, A61M 15/00

(54) **STABLE LIQUID COMPOSITIONS OF GAMMA INTERFERON**
Gamma - Interferon enthaltende stabile wässrige Zusammensetzung
COMPOSITIONS LIQUIDES STABLES D'INTERFERON GAMMA

(30) Priority: 12.05.1993 US 60327
(43) Date of publication of application: 28.02.1996
(73) Proprietor: GENENTECH, INC., South San Francisco California 94080 (US)
(72) Inventor: NGUYEN, Tue, San Mateo, CA 94402 (US)
(74) Representative: Armitage, Ian Michael
(86) International application number: US9404928
(87) International publication number: WO9426302

(56) References cited:
- EP-A- 0 257 956
- WO-A-89/04177

## Description

### BACKGROUND OF THE INVENTION

### 1. Field of the Invention

The present invention concerns stable liquid pharmaceutical compositions comprising gamma interferon (IFN-γ, also known as immune interferon). The invention specifically covers stabilized aqueous pharmaceutical compositions comprising multiple doses of a therapeutically effective amount of IFN-γ for repeated administration.

### II. Description of Background and Related Art

IFN-γ is a member of the interferon family, which exhibits the antiviral and anti-proliferative properties characteristic of interferons-α an -γ (IFN-α and IFN-β) but, in contrast to those interferons, is Ph 2 labile. IFN-γ was originally produced upon mitogenic induction of lymphocytes. The recombinant production of human IFN-γ was first reported by Gray, Goeddel and co-workers [Gray *et al.*, Nature 295, 503-508 (1982)], and is subject of U.S. Patent Nos. 4,762,791, 4,929,544, 4,727,138 and 4,925,793. The recombinant human IFN-γ of Gray and Goeddel as produced in *E. coli*, consisted of 146 amino acids, the N-terminal position of the molecule commencing with the sequence CysTyrCys. It has later been found that the native human IFN-γ (i.e., that arising from mitogen induction of human peripheral blood lymphocytes and subsequent purification) is a polypeptide which lacks the CysTyrCys N-terminus assigned by Gray *et al.*, *supra*. More recently, the crystal structure of *E. coli* derived recombinant human IFN-γ (rhIFN-γ) was determined [Ealick *et al.*, Science 252, 698-702 (1991)] showing that the protein exists as a tightly intertwined non-covalent homodimer, in which the two identical polypeptide chains are oriented in an antiparallel manner.

IFN-γ is known to exhibit a broad range of biological activities, including antitumor, antimicrobial and immunoregulatory activities. Recombinant human IFN-γ (rhifn-γ, Actimmune®, Genentech, Inc. South San Francisco, California) is commercially available as an immunomodulatory drug for the treatment of chronic granulomatous disease characterized by severe, recurrent infections of the skin, lymph nodes, liver, lungs, and bones due to phagocyte disfunction [Baehner, R.L., Pediatric Pathol. 10, 143-153 (1990)]. IFN-γ has also been proposed for the treatment of atopic dermatitis, a common inflammatory skin disease characterized by severe pruritus, a chronically relapsing course with frequent periods of exacerbation, a distinctive clinical morphology and distribution of skin lesions (see PCT Publication No. WO 91/07984 published 13 June 1991), vascular stenosis, including the treatment of restenosis following angioplasty and/or vascular surgery (PCT Publication No. WO 90/03189 published 5 April 1990), various lung conditions, including respiratory distress syndromes (RDS), such as adult respiratory distress syndrome (ARDS) and a neonatal form, termed variously as idiopathic RDS or hyaline membrane disease (PCT Publication No. WO 89/01341 (published 23 February 1989). In addition, IFN-γ has been used with success in the treatment of various allergies, e.g. asthma, and HIV-infection-related conditions, such as opportunistic infections, e.g. *Pneumocystis carinii* pneumonia, and trauma-associated sepsis.

Stable liquid pharmaceutical compositions comprising an effective amount of non-lyophilized IFN-γ along with a buffer capable of maintaining the Ph at 4.0-6.0, a stabilizing agent, such as mannitol, and a non-ionic detergent are disclosed in U.S. Patent No. 5,151,265 issued 29 September 1992.

The known commercial liquid formulation of IFN-γ (Actimmune® rhuIFN-γ-1b, Genentech, Inc.) is a sterile, clear, colorless unpreserved solution filled in a single-dose vial for subcutaneous injection. Each 0.5 ml vial of Actimmune contains 100 µg (3 million U, specific activity: 30 million U/mg) of IFN-γ-1b formulated in 20 mg mannitol, 0.36 mg sodium succinate, 0.05 mg polysorbate 20 and Sterile Water for Injection. As the formulations contain no preservative, they are for one-time use containing a single therapeutic dose, and any unused amount must be discarded.

For certain indications requiring long term use involving repeated administration, such as the treatment of atopic dermatitis or renal cell carcinoma, it would be desirable to develop stable liquid pharmaceutical compositions comprising multiple doses of IFN-γ, in which IFN-γ retains biological activity and physical stability for an extended period of time under recommended storage conditions. Such compositions should preferably contain up to about 30-times of a therapeutically effective amount of IFN-γ for an intended therapeutic use, and remain stable for a least about 14 days following first time administration. However, the preparation of such multi-dose formulations is not at all straightforward.

Proteins, unlike traditional (organic or inorganic) drugs are large in size. It is essential for biological activity that at least a core sequence of their amino acids be preserved intact for conformation integrity. Further, because proteins possess multiple functional groups, such as the various side-chains from their constituent amino acids, the potential exists for many degradation reactions to occur in the same time frame. If such multiple degradation pathways exist, with perhaps different energies of activation, then it is likely that the degradation profile will considerably vary with temperature.

As a result of such complexity, proteins are often unstable, and as they degrade, the determination of the mechanism of their degradation and the characterization of their degradation profile, including the identification of the rate limiting reaction, also is extremely complex. Often there are no straightforward means of determining the chemical identity and measuring the quantity of the degradation products. In general, excipients have a significant effect on the stability of proteins, both in physical terms as well as biochemical, and activity assays, and their careful selection is an important and difficult aspect of formulation design.

The foregoing problems are particularly true for IFN-γ which is an about 15000 D protein, represented by a string of some 143 (146) amino acids, known to be notoriously heat-unstable and prone to aggregation and proteolytic degradation [Wetzel, R.L. *et al.*, "Unfolding and Inactivation" in: Protein Design and the Development of New Therapeutics and Vaccines, Hook, J.B. and Poste, G. eds., Plenum Publishing Corp., 1990, p. 79; Mulkerrin, M.G. and Wetzel, R., Biochemistry 28, 6556 (1989)].

### SUMMARY OF THE INVENTION

The invention concerns a stable aqueous pharmaceutical composition comprising a pharmaceutically effective amount of IFN-γ not subjected to prior lyophilization, an acetate buffer maintaining the Ph within the range of about 4.0 to 6.0, a non-ionic detergent, an isotonifier, and a preservative selected from phenol, benzyl alcohol and a benzethonium halide, e.g. chloride. IFN-γ is preferably present in multiple doses, and retains its biological activity and physical stability without freezing, preferably at 2-8°C, at least up to about two weeks.

In another aspect, the invention concerns a container containing the foregoing liquid pharmaceutical composition in an amount comprising at least one therapeutically effective dose of IFN-γ The container may be a bottle or a vial, or a device containing and capable of disposal of the liquid pharmaceutical composition, such as a syringe or an aerosol container or nebulizer, etc.

In a further aspect, the invention concerns a method of stabilizing IFN-γ in an aqueous formulation by combining previously not lyophilized IFN-γ with water, an acetate buffer maintaining the pH within the range of about 4.0 to 6.0, a non-ionic detergent, an isotonifier and a preservative selected from phenol, benzyl alcohol and a benzethonium halide.

In a still further aspect, the invention concerns a method of intrapulmonary administration of IFN-γ comprising administering to a patient in need by inhalation aerosol particles comprising a pharmaceutically effective amount of IFN-γ not subjected to prior lyophilization, an acetate buffer maintaining the pH within the range of about 4.0 to 6.0, a non-ionic detergent, an isotonifier, and a preservative selected from phenol, benzyl alcohol and a benzethonium halide, wherein the size of the particles is sufficiently small to permit penetration into the alveoli of the lung and from there to the blood stream of a patient.

These and further aspects will be apparent for those skilled in the art.

### BRIEF DESCRIPTION OF THE FIGURES

Figure 1 shows the effect of various preservatives on the bioactivity of rhuIFN-γ (Actimmune®, Genentech, Inc. South San Francisco, California) in an unpreserved liquid pharmaceutical composition, at 25°C.

Figures 2A and 2B show the effect of different concentrations of phenol preservative on the bioactivity of rhuIFN-γ in a liquid pharmaceutical composition, at 25°C.

Figure 3 shows the effect of 0.9% benzyl alcohol on the bioactivity of rhuIFN-γ in liquid formulations containing 1 mM succinate and 5 mM succinate, respectively, at 25°C.

Figure 4 shows the effect of 0.4 % phenol preservative, at 25°C, on the bioactivity of rhuIFN-γ in liquid pharmaceutical compositions, comprising 5 mM succinate and 1 mM succinate, respectively.

Figure 5 shows the effect of phenol on the bioactivity of rhyIFN-γ in a 1 mM succinate vs. 10 mM acetate formulation at 25°C.

### DETAILED DESCRIPTION OF THE INVENTION

In an attempt to provide a multi-dose IFN-γ formulation, in which the remaining pharmaceutically effective doses of IFN-γ are preserved and remain suitable for therapeutic application for an extended period of time following first time administration, the present inventors found that many of the known, pharmaceutically acceptable preservatives are not compatible with other ingredients conventionally used in liquid pharmaceutical formulations of IFN-γ, and that this incompatibility results in a dramatic decline in stability. In particular, they have found that the addition of various therapeutically acceptable preservatives made the commercial liquid pharmaceutical formulation of IFN-γ (Actimmune®, Genentech, Inc.) unstable, resulting in aggregate formation and loss in biological activity. The present invention is the result of successful research producing a stable, preservative-containing aqueous pharmaceutical composition of IFN-γ. As used herein, "gamma interferon", "interferon gamma", and "IFN-γ" are used interchangeably and refer variously to all forms of (human and non-human animal) IFN-γ as are known to be biologically active in accepted IFN-γ assays, such as by inhibition of virus replication in a suitable cell line (inhibition of encephalomyocarditis virus replication in human lung carcinoma cell line A549 for human IFN-γ), induction of class II antigens, heat lability, other antiviral, antitumor or immunoregulatory assays, or neutralization by antibodies having immunoreactivity for IFN-γ but not with IFNs-α and -β, and is meant to include IFN-γ in mature, pro, met, or des(1-3) (also referred to as desCysTyrCys IFN-γ) form, whether obtained from natural source, chemically synthesized or produced by techniques of recombinant DNA technology. A complete description of the preparation of recombinant human IFN-γ (rhuIFN-γ) including its cDNA and amino acid sequences is shown in the United States patents cited hereinabove, such as, for example, U.S. Patent No. 4,762,791. CysTyrCys-lacking recombinant human IFN-γ, including variously truncated derivatives are, for example, disclosed in European Patent Publication No. 146,354. Non-human animal interferons, including IFN-γ, are, for example, disclosed in European Publication No. 88,622. The term includes variously glycosylated forms and other variants and derivatives of such native (wild-type) interferons, whether known in the art or will become available in the future. Examples of such variants are alleles, and the products of site-directed mutagenesis in which residues are deleted, inserted and/or substituted (see, e.g. European Publication No. 146,354 referred to above). IFN-γ is known to have a narrow host range, therefore, IFN-γ homologous to the animal to be treated should be used. In human therapy, the desCysTyrCys variant of the sequence shown in U. S. Patent No. 4,717,138 and its counterpart, EP 77,670, is preferably employed, and optionally the C-terminal variant in which the last four amino acid residues are deleted in post-translational processing.

In a pharmacological sense, in the context of the present invention, a "therapeutically effective amount" of IFN-γ refers to an amount effective in the prevention or treatment of a pathological condition for the treatment of which IFN-γ is effective. Examples of such conditions, without limitation, are various tumors, microbial infections, chronic granulomatous disease, atopic dermatitis, vascular stenosis, including the treatment of restenosis following angioplasty and/or vascular surgery, respiratory distress syndromes (RDS), such as adult respiratory distress syndrome (ARDS) and a neonatal form, termed variously as idiopathic RDS or hyaline membrane disease, allergies, e.g. asthma, and HIV-infection-related conditions, such as opportunistic infections, e.g. *Pneumocystis carinii* pneumonia, tuberculosis, etc.

The term "pharmaceutical composition" refers to preparations which are in such form as to permit the biological activity of the active ingredients to be unequivocally effective, and which contain no additional components which are toxic to the subjects to which the composition would be administered.

"Pharmaceutically acceptable" excipients (vehicles, additives) are those which can reasonably be administered to a subject mammal to provide an effective dose of the active ingredient employed.

IFN-γ "retains its biological activity" in a pharmaceutical composition, if the biological activity at a given time is within about 20% of the biological activity exhibited at the time the pharmaceutical composition was prepared, as determined is a standard IFN-γ bioactivity assay, such as, for example, A549 antiviral bioassay.

IFN-γ "retains its physical stability" in a liquid pharmaceutical composition if it shows no signs of aggregation upon visual examination, or as measured by size exclusion HPLC method.

The 4.0-6.0 pH range has been earlier described as optimal for the stability of IFN-γ in aqueous solution. It has further been shown that upon storage at about 5°C and above, liquid unpreserved formulations comprising previously not lyophilized rhuIFN-γ have considerably better stability than lyophilized rhuIFN-γ formulations, and in particular that in the liquid formulations the aggregate formation is significantly lower. (See, e.g. U.S. Patent No. 5,151,265 issued 29 September 1992.)

It has now been found that the addition of a preservative (antimicrobial agent) to a liquid formulation of IFN-γ in which the pH is maintained by a succinate buffer results in unwanted aggregate formation, and the protein shows a rapid loss of activity in such solutions. In attempting to identify the reasons for the lower stability of the preservative-containing solutions, the present inventors have found that the aggregate formation was not due to the incompatibility of IFN-γ and the preservatives used, rather was caused by the simultaneous presence of the succinate buffer and the preservatives tested. Although the exact nature of the reactions underlying the aggregate formation is not understood, and the degradation products have not been isolated, it was surprisingly found that the degradation could be avoided, and stable aqueous pharmaceutical compositions could be made by replacing the succinate buffer with an acetate buffer capable of maintaining the pH in the desired pH range.

In a first experiment, the effect of various preservatives on the bioactivity of rhuIFN-γ in Actimmune (rhIFN-γ-1b, Genentech, Inc.) was studied. IFN-γ was dialyzed into the buffer of interest. IFN-γ concentration was adjusted to 0.2 mg/ml. Preservatives were added and mixed until dissolved. 1 ml aliquots were transferred into 3 ml Type I glass vials which were stored at 5°C and 25°C. Periodically, vials were selected (one vial/time point/storage temperature) and assayed. Assays included SDS-PAGE, ELISA, A549 antiviral bioassay [see, e.g. Fish, E. N. *et al.*, Drug Design and Delivery 2, 191-206 (1988)], and on a less frequent basis, reverse phase HPLC, ion-exchange and size exclusion chromatography. Only bioactivity and SDS-PAGE data are discussed since they are the most sensitive in reflecting changes. The actual experimental data presented were obtained at 25°C (accelerated stability studies), since at 5°C it would require significantly longer time to notice changes.

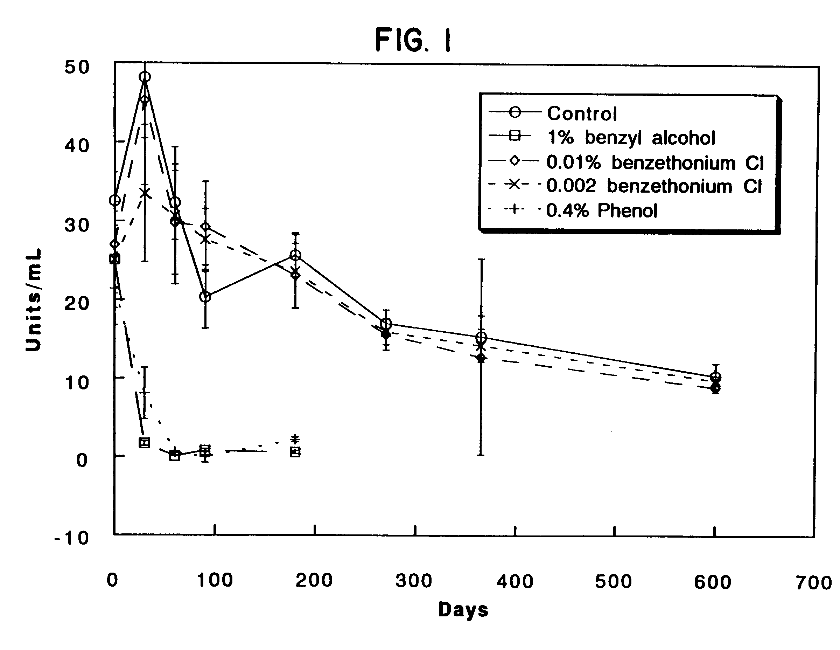

As shown in Figure 1, the addition of 1 % benzyl alcohol or 0.4 % phenol as preservative caused a dramatic decrease in IFN-γ biological activity at 25°C, as compared to the unpreserved control and to formulations comprising two different concentrations of benzethonium chloride.

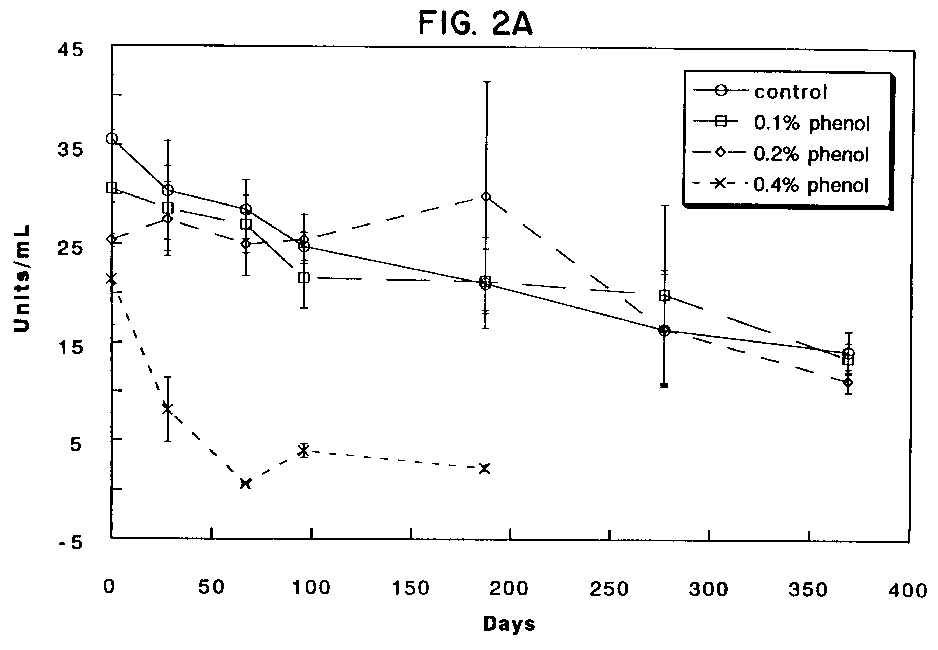

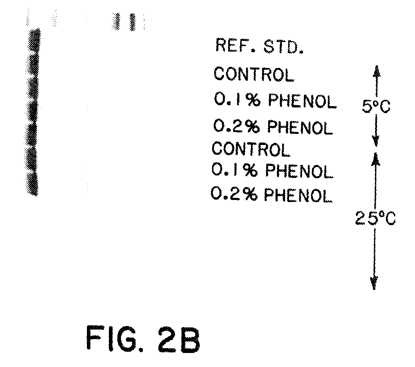

Figure 2A depicts the effect of different concentrations of phenol on the biological activity of rhuIFN-γ (Actimmune®, Genentech, Inc.) in liquid formulation at 25°C. Although the IFN-γ bioactivity was satisfactory in the solutions preserved with 0.1% and 0.2 % phenol, respectively, aggregate formation was increased in these solutions, as tested by Sodium Dodecyl Sulfate Polyacrylamide Gel Electrophoresis (SDS-PAGE) (Figure 2B).

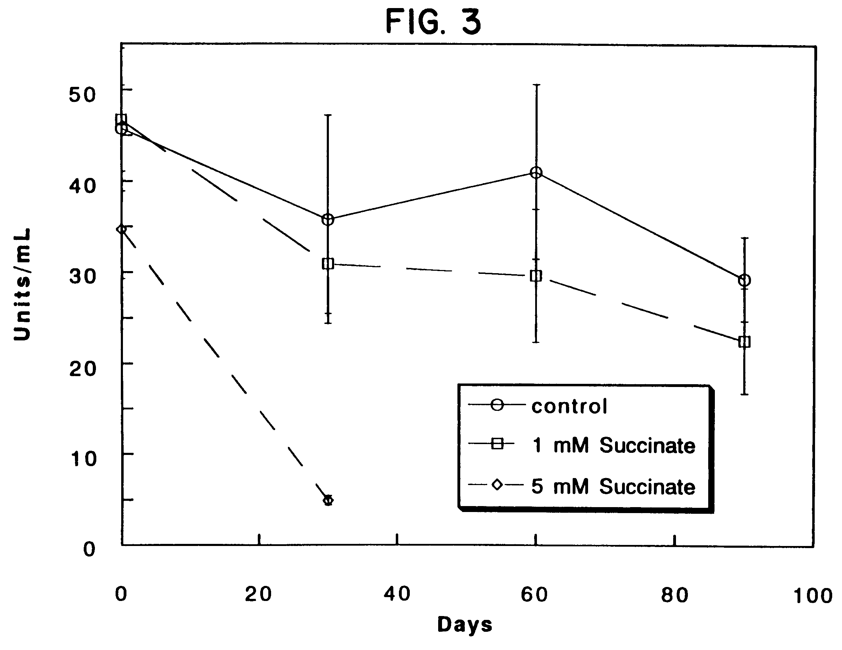

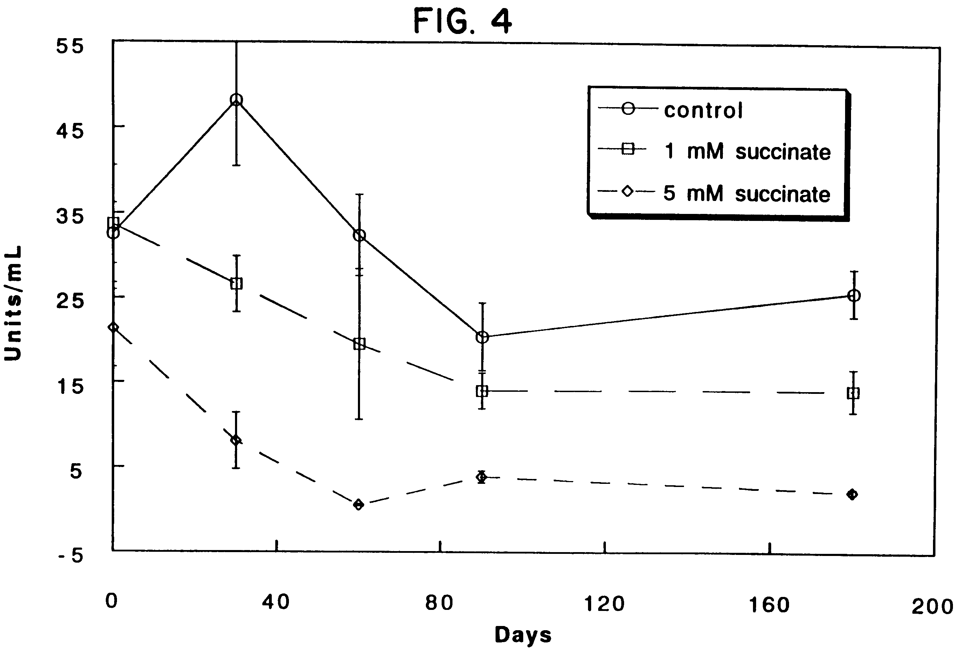

Figure 3 illustrates the effect of 0.9 % benzyl alcohol on rhuIFN-γ bioactivity at 25°C in formulations buffered with 1 mM succinate and 5 mM succinate, respectively. The fact that the loss in bioactivity is less in the formulation containing a lower concentration of succinate appears to indicate that the presence of succinate buffer is at least partially responsible for the stability problems experienced with preserved rhuIFN-γ solutions. This conclusion is supported by the results shown in Figure 4. The loss in IFN-γ biological activity was less dramatic in aqueous solutions the pH of which was maintained at 5.0 with 1 mM succinate than in those containing 5 mM succinate.

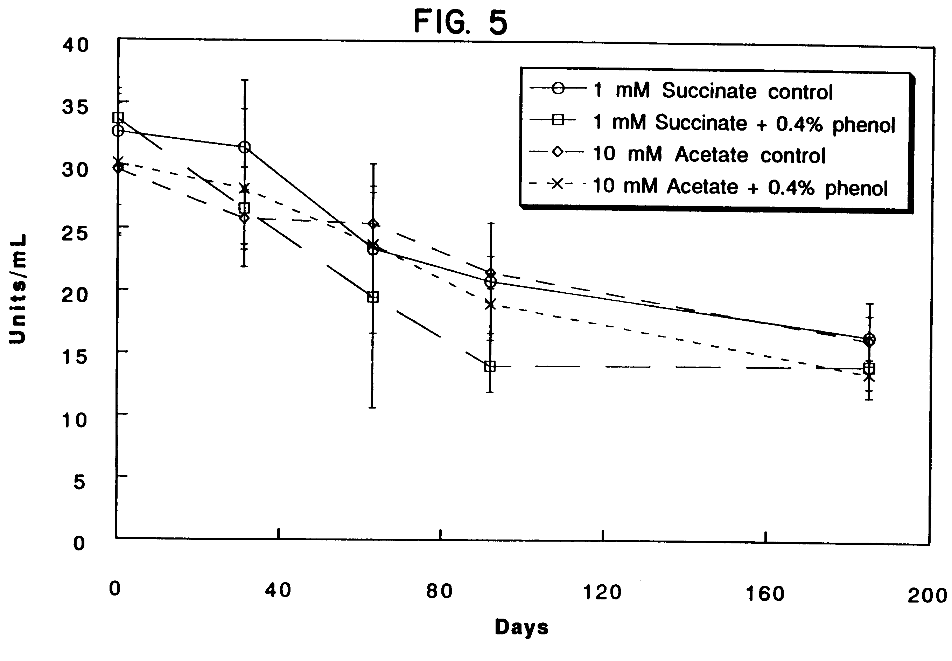

The effect of 0.4 % phenol on the bioactivity of rhuIFN-γ was then studied in liquid formulations comprising 1 mM succinate buffer and 10 mM acetate buffer, respectively. As shown in Figure 5, the stability of acetate-buffered solution, judged by IFN-γ biological activity, was essentially the same as those of the unpreserved control solutions buffered with succinate and acetate, respectively, and no aggregates were detected by visual evaluation or SDS-PAGE electrophoresis.

The pharmaceutical compositions of the present invention contain:
a) IFN-γ not subjected to prior lyophilization;
b) an acetate buffer capable of maintaining the pH between about 4 and about 6 (the pH range of maximum stability of the protein in solution);
c) a non-ionic detergent primarily to stabilize the protein against agitation-induced aggregation;
d) an isotonifier;
e) a preservative selected from the group of phenol, benzyl alcohol and a benzethonium halide, e.g. chloride; and
f) water.

The non-ionic detergents (surfactants) may, for example, be polysorbates (e.g. polysorbate (Tween®) 20, 80, etc.) or poloxamers (e.g. poloxamer 188). The use of non-ionic surfactants permits the formulation to be exposed to shear surface stresses without causing denaturation of the protein. Further, such surfactant containing formulations may be employed in aerosol devices such as those used in a pulmonary dosing, and needleless jet injector guns (see, e.g. EP 257,956).

The isotonifier is present to ensure isotonicity of the liquid compositions of the present invention, and includes polyhydric sugar alcohols, preferably trihydric or higher sugar alcohols, such as glycerin, erytritol, arabitol, xylitol, sorbitol and mannitol. These sugar alcohols can be used alone or in combination. Alternatively, sodium chloride or other appropriate inorganic salts may be used to render the solutions isotonic.

The acetate buffer may, for example, be an acetic acid-sodium acetate mixture, acetic acid-sodium hydroxide mixture, etc. The pH of the liquid formulation of this invention is buffered in the range of about 4.0 to 6.0, preferably 4.5 to 5.5, and most preferably at about pH 5.

The preservatives phenol, benzyl alcohol and benzethonium halides, e.g. chloride, are known antimicrobial agents.

In a preferred embodiment, the liquid pharmaceutical composition of the present invention comprises the following components:
- IFN-γ: 0.1-2.0 mg/ml
- sodium acetate (pH 5.0): 5-100 mM
- Tween 20: 0.1 to 0.01 % by weight
- phenol: 0.05 to 0.4 % by weight
- mannitol: 5 % by weight
- water for injection, USP: up to 100 %,
wherein the percentage amounts are based on the weight of the composition. Phenol can be replaced by 0.5-1.0 % by weight of benzyl alcohol, and mannitol can be replaced by 0.9 % by weight sodium chloride.

Most preferably, the compositions comprise
- IFN-γ: 0.1 to 1.0 mg/ml
- sodium acetate (pH 5.0): 10 mM
- Tween 20: 0.01 % by weight
- phenol: 0.2 %
- mannitol: 5 %

Phenol can be replaced by 0.75 by weight benzyl alcohol and mannitol by 0.9 % by weight sodium chloride.

The preserved liquid formulations preferably contain multiple doses of a therapeutically effective amount of IFN-γ. In view of the narrow host range of this polypeptide, for the treatment of human patients liquid formulations comprising human IFN-γ, more preferably native sequence human IFN-γ, are preferred. As a biological response modifier, IFN-γ exerts a wide variety of activities on a wide range of cell types, in a variety of human and non-human mammalian species. The therapeutically effective dose will, of course, vary depending on such factors as the pathological condition to be treated (including prevention), the patient's age, weight, general medical condition, medical history, etc., and its determination is well within the skill of a practicing physician. The effective dose generally is within the range of from about 0.001 to about 1.0 mg/kg, more preferably about 0.01-1 mg/kg, most preferably about 0.01-0.1 mg/kg. In such formulations huIFN-γ will preferably exhibit a specific activity of on the order of about 2 x 10⁷ U/mg of protein or greater when tested on A549 cells against encephalomyocarditis virus. It should be appreciated that endotoxin contamination should be kept minimally at a safe level, for example, less than 0.5 ng/mg protein. Moreover, for human administration, the liquid formulations should meet sterility, pyrogenicity, general safety, and purity as required by FDA Office and Biologics standards.

The formulations are preferably administered as repeated intravenous (i.v.), subcutaneous (s.c.) or intramuscular (i.m.) injections, or as aerosol formulations suitable for intranasal or intrapulmonary delivery (for intrapulmonary delivery see, e.g. EP 257,956).

For the treatment of patients with chronic granulomatous disease the recommended dosage is 50 µg/m² (1.5 million U/m²) for patients whose body surface area is greater than 0.5 m², and 1.5 µg/kg/dose for patients whose body surface area is equal to or less than 0.5 m². Injection is recommended to be administered subcutaneously three times weekly.

For reducing infection and death in patients sustaining severe injury, a dose of 100 µg rhuIFN-γ can be administered subcutaneously once daily. Infections that can be prevented or treated this way include pneumonia, bacteremia, intra-abdominal or intrathoracic infection, major wound infection, vecticulitis/meningitis, etc.

The stable aqueous compositions of the present invention are preferably contained in vials, containing up to about 30 therapeutically effective doses of IFN-γ. The bioactivity of IFN-γ preferably remains within about 20 % from the bioactivity exhibited at the time of first administration for at least about 14 days, more preferably for at least about 200 days following first administration.

All citations throughout the specification, and the references cited therein, are hereby expressly incorporated by reference.

Although the foregoing refers to particular preferred embodiments, it will be understood that the present invention is not so limited. It will occur to those ordinarily skilled in the art that various modifications may be made to the disclosed embodiments within the scope of the present invention.

## Claims

1. A stable aqueous pharmaceutical composition comprising a pharmaceutically effective amount of IFN-γ not subjected to prior lyophilization, an acetate buffer maintaining the pH between 4.0 and 6.0, a non-ionic detergent, an isotonifier, and a preservative selected from the group consisting of phenol, benzyl alcohol and a benzethonium halide.

2. The composition to claim 1 wherein IFN-γ is human IFN-γ.

3. The composition of claim 2 wherein IFN-γ is desCysTyrCys-IFN-γ.

4. The composition of claim 2 wherein IFN-γ is desCysTyrCys-IFN-γ from which the four C-terminal amino acids have been deleted.

5. The composition of any one of the preceding claims wherein the specific activity of IFN-γ is at least 2 x 10⁷ U/mg.

6. The composition of any one of the preceding claims comprising at least two therapeutically effective doses of human IFN-γ.

7. The composition of claim 6 comprising 2 to 30 therapeutically effective doses of human IFN-γ.

8. The composition of any one of the preceding claims wherein the non-ionic detergent is a polysorbate.

9. The composition of any one of the preceding claims wherein the isotonifier is a polyhydric sugar alcohol.

10. The composition of any one of the preceding claims wherein the polyhydric sugar alcohol is mannitol.

11. The composition of any one of the preceding claims wherein the preservative is phenol or benzyl alcohol.

12. A container containing a stable aqueous pharmaceutical composition comprising a pharmaceutically effective amount of IFN-γ not subjected to prior lyophilization, an acetate buffer maintaining the pH between 4.0 and 6.0, a non-ionic detergent, an isotonifier, and a preservative selected from the group consisting of phenol, benzyl alcohol and a benzethonium halide.

13. The container of claim 12 which is a vial comprising at least two doses of a therapeutically effective amount of IFN-γ.

14. The container of claim 12 or claim 13 which is capable of disposal of the contained composition by injection or by aerosol delivery.

15. A method of stabilising IFN-γ in an aqueous formulation by combining previously not lyophilized IFN-γ with water, an acetate buffer maintaining the pH between 4.0 and 6.0, a non-ionic detergent, an isotonifier and a preservative selected from phenol, benzyl alcohol and a benzethonium halide.

16. The composition of any one of claims 1 to 11 for use in a method of medical treatment.

17. The use of a composition comprising a pharmaceutically effective amount of IFN-γ not subjected to prior lyophilization, an acetate buffer maintaining the pH between 4.0 and 6.0, a non-ionic detergent, an isotonifier agent, and a preservative selected from phenol, benzyl alcohol and a benzethonium halide in the preparation of a medicament for the intrapulmonary administration of IFN-γ wherein the size of the particles is sufficiently small to permit penetration into the alveoli of the lung and from there to the blood stream of a patient.

## Patentansprüche

1. Stabile, wäßrige, pharmazeutische Zusammensetzung, umfassend eine pharmazeutisch wirksame Menge von IFN-γ, das zuvor keiner Lyophilisierung unterzogen wurde, einen Acetatpuffer, der den pH-Wert zwischen 4,0 und 6,0 hält, ein nicht-ionogenes Detergens, ein Isotonisierungsmittel und ein Konservierungsmittel, ausgewählt aus der Gruppe bestehend aus Phenol, Benzylalkonol und einem Benzethoniumhalogenid.

2. Zusammensetzung nach Anspruch 1, worin IFN-γ menschliches IFN-γ ist.

3. Zusammensetzung nach Anspruch 2, worin das IFN-γ desCysTyrCys-IFN-γ ist.

4. Zusammensetzung nach Anspruch 2, worin das IFN-γ desCysTyrCys-IFN-γ ist, aus dem die vier C-terminalen Aminosäuren deletiert wurden.

5. Zusammensetzung nach einem der vorhergehenden Ansprüche, worin die spezifische Aktivität von IFN-γ zumindest 2 x 10⁷ U/mg beträgt.

6. Zusammensetzung nach einem der vorhergehenden Ansprüche, umfassend zumindest zwei therapeutisch wirksame Dosen von menschlichem IFN-γ.

7. Zusammensetzung nach Anspruch 6, umfassend 2 bis 30 therapeutisch wirksame Dosen von menschlichem IFN-γ.

8. Zusammensetzung nach einem der vorhergehenden Ansprüche, worin das nicht-ionogene Detergens Polysorbat ist.

9. Zusammensetzung nach einem der vorhergehenden Ansprüche, worin das Isotonisierungsmittel ein mehrwertiger Zuckeralkohol ist.

10. Zusammensetzung nach einem der vorhergehenden Ansprüche, worin der mehrwertige Zuckeralkohol Mannit ist.

11. Zusammensetzung nach einem der vorhergenenden Ansprüche, worin das Konservierungsmittel Phenol oder Benzylalkohol ist.

12. Behälter, der eine stabile, wäßrige, pharmazeutische Zusammensetzung enthält, umfassend eine pharmazeutisch wirksame Menge von IFN-γ, das vorher keiner Lyophilisierung unterzogen wurde, einen Acetatpuffer, der den pH-Wert zwischen 4,0 und 6,0 hält, ein nicht-ionogenes Detergens, ein Isotonisierungsmittel und ein Konservierungsmittel, ausgewählt aus der Gruppe bestehend aus Phenol, Benzylalkohol und einem Benzethoniumhalogenid.

13. Behälter nach Anspruch 12, der ein Fläschchen ist, das zumindest zwei Dosen einer therapeutisch wirksamen Menge von IFN-γ umfaßt.

14. Behälter nach Anspruch 12 oder 13, der zur Verabreichung der enthaltenen Zusammensetzung durch Injektion oder durch Aerosolabgabe fähig ist.

15. Verfahren zum Stabilisieren von IFN-γ in einer wäßrigen Formulierung durch Zusammenbringen von zuvor nicht lyophilisiertem IFN-γ mit Wasser, einem Acetatpuffer, der den pH-Wert zwischen 4,0 und 6,0 hält, einem nicht-ionogenen Detergens, einem Isotonisierungsmittel und einem Konservierungsmittel, das aus Phenol, Benzylalkohol und einem Benzethoniumhalogenid ausgewählt ist.

16. Zusammensetzung nach einem der Ansprüche 1 bis 11 zur Verwendung in einem Verfahren zur medizinischen Behandlung.

17. Verwendung einer Zusammensetzung, umfassend eine pharmazeutisch wirksame Menge von IFN-γ, das zuvor keiner Lyophilisierung unterzogen wurde, einen Acetatpuffer, der den pH-Wert zwischen 4,0 und 6,0 hält, ein nicht-ionogenes Detergens, ein Isotonisierungsmittel und ein Konservierungsmittel, ausgewählt aus Phenol, Benzylalkohol und einem Benzethoniumhalogenid, zur Herstellung eines Medikaments zur intrapulmonären Verabreichung von IFN-γ, worin die Teilchengröße ausreichend klein ist, um das Eindringen in die Lungenbläschen und von dort in den Blutstrom eines Patienten zu ermöglichen.

## Revendications

1. Composition pharmaceutique aqueuse stable comprenant une quantité pharmaceutiquement efficace de IFN-γ non soumise à une lyophilisation préalable, un tampon a l'acétate maintenant le pH dans la plage de 4,0 à 6,0, un détergent non ionique et un agent d'isotonicité, ainsi qu'un conservateur choisi dans le groupe consistant en phénol, alcool benzylique et un halogénure de benzéthonium.

2. Composition suivant la revendication 1, dans laquelle le IFN-γ consiste en le INF-γ humain.

3. Composition suivant la revendication 2, dans laquelle le IFN-γ consiste en desCysTyrCys-INF-γ.

4. Composition suivant la revendication 2, dans laquelle le INF-γ consiste en desCysTyrCys-INF-γ dont les quatre amino-acides C-terminaux ont été éliminés par délétion.

5. Composition suivant l'une quelconque des revendications précédentes, dans laquelle l'activité spécifique du IFN-γ est égale à au moins 2 x 10⁷ U/mg.

6. Composition suivant l'une quelconque des revendications précédentes, comprenant au moins deux doses thérapeutiquement efficaces de INF-γ humain.

7. Composition suivant la revendication 6, comprenant 2 à 30 doses thérapeutiquement efficaces de IFN-γ humain.

8. Composition suivant l'une quelconque des revendications précédentes, dans laquelle le détergent non ionique est un polysorbate.

9. Composition suivant l'une quelconque des revendications précédentes, dans laquelle l'agent d'isotonicité est un sucre-alcool polyhydroxylique.

10. Composition suivant l'une quelconque des revendications précédentes, dans laquelle le sucre-alcool polyhydroxylique est le mannitol.

11. Composition suivant l'une quelconque des revendications précédentes, dans laquelle le conservateur est le phénol ou l'alcool benzylique.

12. Récipient contenant une composition pharmaceutique aqueuse stable comprenant une quantité pharmaceutiquement efficace de INF-γ non soumise à une lyophilisation préalable, un tampon à l'acétate maintenant le pH dans la plage de 4,0 à 6,0, un détergent non ionique, un agent d'isotonicité, et un conservateur choisi dans le groupe consistant en phénol, alcool benzylique et un halogénure de benzéthonium.

13. Récipient suivant la revendication 12, qui consiste en un flacon comprenant au moins deux doses d'une quantité thérapeutiquement efficace de INF-γ.

14. Récipient suivant la revendication 12 ou la revendication 13, qui est apte à la distribution de la composition qu'il renferme par injection ou par émission d'un aérosol.

15. Procédé pour stabiliser du IFN-γ dans une formulation aqueuse en associant du INF-γ préalablement non lyophilisé avec de l'eau, un tampon à l'acétate maintenant le pH dans la plage de 4,0 à 6,0, un détergent non ionique, un agent d'isotonicité et un conservateur choisi entre le phénol, l'alcool benzylique et un halogénure de benzéthonium.

16. Composition suivant l'une quelconque des revendications 1 à 11, destinée à être utilisée dans une méthode de traitement médical.

17. Utilisation d'une composition comprenant une quantité pharmaceutiquement efficace de INF-γ non soumise à une lyophilisation préalable, un tampon à l'acétate maintenant le pH dans la plage de 4,0 à 6,0, un détergent non ionique, un agent d'isotonicité et un conservateur choisi entre le phénol, l'alcool benzylique, un halogénure de benzéthonium, dans la préparation d'un médicament pour l'administration intrapulmonaire du IFN-γ, dans laquelle le diamètre des particules est suffisamment petit pour permettre une pénétration dans les alvéoles du poumon, et à partir de ces alvéoles, dans le courant sanguin d'un patient.
